(19) Europäisches Patentamt
European Patent Office
Office européen des brevets

(11) Publication number: **0 259 843**
**A2**

(12) # EUROPEAN PATENT APPLICATION

(21) Application number: **87113131.4**

(51) Int. Cl.$^4$: **A61K 7/15**

(22) Date of filing: **08.09.87**

(30) Priority: **09.09.86 US 905592**

(43) Date of publication of application:
**16.03.88 Bulletin 88/11**

(84) Designated Contracting States:
**AT BE CH DE ES FR GB GR IT LI LU NL SE**

(71) Applicant: **S.C. JOHNSON & SON, INC.**
**1525 Howe Street**
**Racine, Wisconsin 53403(US)**

(72) Inventor: **Jaynes, Edgar N.**
**405 Lombard Avenue**
**Racine Wisconsin 53402(US)**
Inventor: **Leskowicz, James J.**
**3605 Lathrop Avenue**
**Racine Wisconsin 53405(US)**

(74) Representative: **Baillie, Iain Cameron et al**
**c/o Ladas & Parry Isartorplatz 5**
**D-8000 München 2(DE)**

(54) **Post-foaming gel with an unsaturated or non-linear dialkanolamide or a monoalkanolamide.**

(57) A novel post-foaming gel includes an effective amount of a water-soluble soap, a post-foaming agent, a gelling aid and an unsaturated or non-linear alkanolamide or a linear monoalkanolamide in a water base.

EP 0 259 843 A2

The present invention relates to post-foaming compositions suitable for use as cleansing or cosmetic compositions. More particularly, this invention relates to improved post-foaming shave cream compositions.

Prior art post-foaming gels commonly use propylene glycol isostearate or propylene glycol dipelargonate esters. These esters provide the gel with better lubricity and provide a clearer and stronger gel. U. S. Patent 3,541,581 describes and claims such post-foaming gel compositions, including compositions which are suitable for use as shaving preparations. Unfortunately, the esters are expensive and, also, it has been determined that the esters do not function well with most stearic and palmitic fatty acid soap mixtures.

In U. K. Patent No. 1,444,334 it has been proposed to employ saturated long-chain ($C_{12}$-$C_{22}$) amido dialkanol amines to aid in providing a clear, esthetically pleasing product. In addition, the label of a commercial post-foaming gel shave cream has recited use of lauramide diethanolamine (lauramide DEA), as a fatty diethanolamide, among its ingredients. While such fatty dialkanolamides, as myristic diethanolamide, stearamide diethanolamine, cocamide diethanolamide and lauramide diethanolamine have been postulated for use in post-foaming gels, they do not impart the levels of clarity or gel strength which are desired for such products. Therefore, it would be of particular economic benefit to be able to replace the post-foaming gel's esters with less expensive compounds, particularly compounds which are more compatible with stearic and palmitic soaps and which impart enhanced clarity and strength to such gels.

Accordingly, the object of this invention is to provide a post-foaming gel which does not require an ester substituent for clarity and strength.

The present invention provides an aqueous post-foaming gel composition which includes a water-soluble soap, a volatile post-foaming agent and a water-soluble polymer gelling agent, characterized by the composition also including an alkanolamide having the formula:

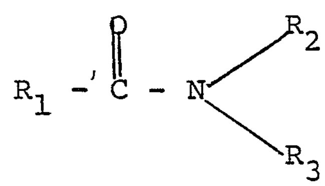

wherein $R_1$ is selected from the group consisting of non-linear $C_{10}$-$C_{22}$ fatty alkyls, $R_2$ is H or a $C_1$-$C_4$ hydroxyalkyl and $R_3$ is a $C_1$-$C_4$ hydroxyalkyl provided that if $R_1$ is a linear $C_{10}$-$C_{22}$ fatty alkyl, then $R_2$ is H to enhance clarity and gel strength.

The composition of this invention has enhanced compatibility with adjuvant materials and has a broader range of lathering and conditioning characteristics. The composition of the present invention has superior performance at a lower cost and is particularly suited to packaging in conventional aerosol dispensing containers.

The invention will now be described in greater detail.

The alkanolamides useful in the present invention have the structure as set forth in formula I, where $R_1$ is selected from the group consisting of linear $C_{10}$-$C_{22}$ fatty alkyls, unsaturated $C_{10}$-$C_{22}$ fatty alkyls, and non-linear $C_{10}$-$C_{22}$ fatty alkyls, $R_3$ is a $C_1$-$C_4$ hydroxyalkyl group and $R_2$ is H or a $C_1$-$C_4$ hydroxyalkyl where if $R_1$ is a linear $C_{10}$-$C_{22}$ fatty alkyl, then R2 is H.

Formula (I)

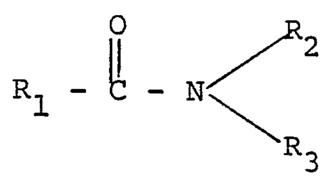

Preferred fatty alkanolamides include: Lauramide monoisopropylamine; linoleamide diethanolamine; oleamide diethanolamine; isostearamide diethanolamine; tall oil diethanolamide and lauramide monoethanolamine.

If desired, various fatty diethanolamides, such as lauramide diethanolamine, myristic diethanolamine, stearamide diethanolamine, cocamide diethanolamine and the like may be admixed with the alkanolamides.

The alkanolamide is preferably employed in amounts such that the alkanolamide concentration is at least about 5 percent by weight of the water soluble soap concentration. This is generally true regardless of the CTFA designation of the particular alkanolamide used. Preferably, the alkanolamide is employed in amounts from about 1 to 6 percent by weight of the total composition.

Water provides the desired foaming and lathering qualities and also properly moistens the face and beard for proper shaving. Water from any source, including tap water, distilled water, deionized water and the like may be beneficially used in the present invention. In general, water is preferably present in the amount of 60-90% by weight of the total composition, in order to best realize the desired degree of fluidity and mobility in the post-foaming gel product.

The water-soluble soap is preferably a water-soluble salt of a higher fatty acid. Such soaps are well known to the art and may be purchased or prepared in any conventional manner. For example, these soaps may be prepared by reacting a basic material such as triethanolamine directly with a fatty acid such as a saturated or unsaturated fatty acid which is C10 to C22, or various mixtures of these acids. Preferred soaps include the water-soluble stearate and palmitate soaps, such as the soluble amine soaps of commercial stearic acid and palmitic acid. The triethanolamine soaps of these acids are especially preferred. The water-soluble soap is preferably employed in amounts from about 5 to 20% by weight of the total composition.

It is well known that the commercial product known as stearic acid is actually a mixture consisting primarily of stearic and palmitic acids. Therefore, the term "stearates" is used herein to designate soaps of commercial stearic acid, although soaps of chemical pure stearic or palmitic acid work equally well for the purposes of this invention.

The preferred gelling aids, found to be most suitable for use in the present invention, are water-soluble hydroxyalkyl cellulose or naturally derived gums such as xanthan,. various synthesized polymers such as polyvinyl pyrrolidone, as well as chemically or enzymatically modified derivatives of these materials. These materials contribute sufficient viscosity to provide the desired levels of lubricity and stability to the product. Hydroxyalkyl cellulose thickeners may be commonly produced by combining an alkyl cellulose and an alkylene oxide such as ethylene or propylene oxide. Products of this type are marketed under the trademarks "Natrosol" and "Klucel" and are available in a variety of viscosity grades. One such preferred grade of hydroxyalkyl cellulose is the hydroxyethyl cellulose sold under the trademark "Natrosol 250 HHR".

Other naturally occurring polysaccharide polymers and their derivatives, as well as synthesized polymers, such as polyvinyl pyrrolidone, can be used to provide equivalent performance in the formulation.

The exact amount of gelling aid used in the practice of the invention will depend to some extent upon the viscosity grade employed, but will generally be on the order of about 0.1-5% by weight as based on the total weight of the composition. Mixtures of two or more gelling aids can be employed.

The post-foaming agents used are liquids or are liquifiable and include saturated aliphatic hydrocarbons having from 4 to 6 carbon atoms, including butanes, such as n-butane or isobutane; pentanes such as n-pentane, or isopentane; and hexane. Mixtures of hydrocarbon post-foaming agents may be especially useful for providing the particular vapor pressure desired. An advantage of using mixtures of two or more post-foaming agents is that it permits precise, accurate adjustment of the vapor pressure of the gel to achieve the desired foaming qualities. The addition of the hydrocarbon post-foaming agent to the premixed blend of the other ingredients changes the cloudy soap intermediate to a clear gel and also increases the viscosity of the product.

The concentration of the post-foaming agent in the gel is preferably from about 0.5 to 10% by weight of the total composition. Substantially smaller amounts of post-foaming agent will fail to provide the desirable foaming and lathering characteristics. Significantly larger amounts may cause premature foaming of the composition as it is being expelled from the container.

The range of useful propellent combinations used in this teaching is broader than the range possible with previous teachings, and allows the production of a broader range of foam characteristics than previously possible.

Adjuvant materials are compatible with these formulations. Examples of compatible materials include, but are not limited to, fatty alcohols, aliphatic fatty acid esters, lanolin, lanolin alcohols, glycerides, mineral oil, and many others. Coloring agents, dyes, perfumes, and fragrance oils may also be added. In many cases, however, increased amounts of the alkanolamides of the invention may be required to provide clear, firm gels in the presence of such added material.

As indicated above, the addition of various adjuvant materials to the stable gel is contemplated by the present invention. Thus, the finished commercial compositions will ordinarily contain materials such as, but not limited to, dyes, perfumes, humectants, natural oils, esters, glycerine, lanolin, aloe, lecithin, fatty alcohols, OTC Monograph active materials, nonionic surfactants, amphoterics, polymers, insoluble particulates, or other materials which provide particularly desired properties.

The following examples are illustrative of compositions falling within the general scope of the invention. The following compositions were prepared and evaluated as shaving preparations. All amounts are given in parts by weight.

EXAMPLE I

In order to prepare a series of post-foaming gels within the scope of this invention the following procedure was employed.

A fatty acid and an alkanolamide of the invention were mixed and preheated to 82.2° C. (180°F.). The dye and Natrosol are blended into 15.5°C. (60°F.) water. The water mixture is heated to 82.2°C. (180°F.) and the amine is added. The amide acid mixture at 82.2°C. (180°F.) is added to the water mixture at 82.2°C. (180°F.) with agitation. After mixing, the resulting mixture is cooled to 71.1°C. (160°F.). At this point, other desired additives were introduced. Solid additive materials were warmed to 60°C. (140°F.) before being added to the mixture. The mixture was then cooled to 37.7°C. (100°F.) and any desired perfumes were added.

After any necessary mixing was completed, the mixture was further cooled to 4.4°C. (40°F.). This mixture was then mixed with the particular hydrocarbon blend and filled into an aerosol dispenser wherein the mixture is separated from the propellent such as by a collapsible bag or diaphragm such as that described in U. S. Patent 3,542, 581. Table A lists representative prepared compositions.

TABLE A

| Ingredient | Amount (% by weight) 1 | 2 | 3 | 4 |
|---|---|---|---|---|
| Deionized Water | 81.65 | 81.65 | 75.27 | 79.95 |
| *Natrosol 250HHR | 0.35 | 0.35 | 0.50 | 0.45 |
| Triethanolamine U.S.P. (85%) | 5.58 | 5.58 | 7.36 | 6.00 |
| *Emersol 132 | 9.92 | 9.92 | 11.87 | 10.60 |
| *Clindrol 102 LI | 2.50 | ---- | ---- | 1.00 |
| *Clindrol LT 15-73-1 | ---- | 2.50 | 2.33 | 1.50 |
| *Clindrol CP1-24-2 | ---- | ---- | 2.67 | ---- |
| Lauryl Alcohol | ---- | ---- | ---- | 0.50 |
| Dye | q.s. | q.s. | q.s. | q.s. |
| Perfume | q.s. | q.s. | q.s. | q.s. |
| Pressurized Formulation: | 1 | 2 | 3 | 4 |
| Intermediate (from above) | 97.52 | 97.52 | 97.52 | 97.52 |
| n-Pentane | 1.73 | 1.73 | 1.73 | 1.73 |
| Isobutane | 0.75 | 0.75 | 0.75 | 0.75 |

*Natrosol 250 HHR - Hydroxyethyl cellulose
*Emersol 132 - Triple pressed stearic acid
*Clindrol 102 LI - Lauramide MIPA
*Clindrol LT 15-73-1 - Linoleamide DEA
*Clindrol CP1-24-2 - Lauramide DEA/Linoleamide DEA, 2: 1

The resulting products are highly satisfactory shaving preparations which are expelled from the can as a gel and which thereafter form a rich lather when spread on the face.

## EXAMPLE 2

The post-foaming gels of the invention containing the alkanolamides were compared to post-foaming gels containing saturated fatty acid dialkanolamides to evaluate their improved gel strength and clarity.

Gel clarity and strength were determined as follows. Gel clarity was determined by filling a 2.5 cc disposable syringe with the finished product directly from the can and by visually assigning a clarity number, as described in Table B.

TABLE B

| Number | Description | Property |
|---|---|---|
| 5 | Clear | Transparent |
| 4 | Semi-Clear | Syringe markings easily read. |
| 3 | Translucent | Syringe markings appear fuzzy. |
| 2 | Hazy | Syringe markings barely discernible. |
| 1 | Cloudy | Unable to see markings. |

Clarity was also assessed by dispensing a can of material into a flow cell mounted in a spectrophotometer. A wavelength was chosen where dye absorbance was low. The percent transmittance of various formulations which were measured at that wavelength by this method correlated extremely well with the simpler visual evaluation described above.

Gel strength was assessed by use of a tine tester in which metal rods were arranged horizontally in comb fashion such that each pair of rods was separated by an increasingly large distance. The rods thereby form a grid onto which finished products were actuated. A stronger gel will naturally span a larger number of tines.

Foaming speed was also assessed during this test, since the tines are only counted after sixty seconds of equilibration. If the product in question has foamed or expanded in size by more than 10% of its original volume, the measured number of tines is not a good representation of gel strength. Gels that foam in less than 60 seconds are characterized as weak, since they are unable to resist the force of the expanding post-foaming agent.

In order to assess the gel strength and clarity of composition of this invention, eight formulations were prepared in accordance with the procedure of Example 1. The formulations were identical in constituents and amounts to formulation 2 in Table A of Example 1, except that the following alkanolamides were substituted for linoleamide diethanolamine (Clindrol LT 15-73-1), as follows:

TABLE C

| Alkanolamide | Clarity | % T(1) | Strength (2) |
|---|---|---|---|
| Clindrol LT 15-73-1 | 5 | 26 | 18+ |
| Monamid 150 IS (3) | 4 | 23 | 18+ |
| Monamid 150 MW (4) | 2 | 1.0 | 16 |
| Clindrol Superamide 100S (5) | 1 | 0.3 | 9 |
| Clindrol 102-LI (6) | 4 | 16 | 18+ |
| Clindrol Superamide 100C (7) | 2 | 1.2 | 8 |
| Monamine T-100 (8) | 5 | 53 | 18+ |
| Clindrol 200-L (9) | 3 | 15 | 6 |
| Monamid LMA (10) | 5 | 30 | 18+ |

---------------------------

(1) Percent transmittance: 530nm, 1cm cell, 75°F.

(2) Tine tester constructed with 18 tines maximum: average of two strips, 60 sec.

(3) Isostearamide DEA - inventive formulation.

(4) Myristic DEA - comparative formulation.

(5) Stearamide DEA - comparative formulation.

(6) Lauramide MIPA - inventive formulation.

(7) Cocamide DEA - comparative formulation

(8) Tall Oil DEA - inventive formulation.

(9) Lauramide DEA - comparative formulation.

(10) Lauramide MEA - inventive formulation.

As illustrated above, the compositions of the present invention are highly satisfactory shaving preparations which exhibit clear gels having high gel strength.

The following formulation was prepared according to the procedure of Example I.

| Ingredient | % by Weight |
|---|---|
| Deionized water | 78.669 |
| D&C Yellow #10, 0.1% | q.s. |
| FD&C Blue #1, 0.12 | q.s |
| Natrosol 250 HHR (1) | 0.450 |
| Triethanolamine, 85% | 6.000 |
| Emersol 132 Stearic Acid (2) | 10.600 |
| Clindrol 202-0 (3) | 3.250 |
| Perfume | q.s. |
| | 100.000% |

(1) Hydroxyethyl cellulose, high molecular weight.

(2) Triple pressed stearic acid; 50% stearic acid; 48% palmitic acid.

(3) Oleamide DEA, approximately 75% oleic acid side chains.

The above formulation was a good gel shave product having good gel clarity and gel strength.

It should be understood that various modifications can be made to the preferred embodiments disclosed herein without departing from the spirit and scope of the invention, or without the loss of its attendant advantages. Thus, other examples applying the principles described herein are intended to fall within the scope of the invention.

## Claims

1. An aqueous post-foaming gel composition which includes a water-soluble soap, a volatile post-foaming agent and a water-soluble polymer gelling agent, characterized by the composition also including an alkanolamide having the formula:

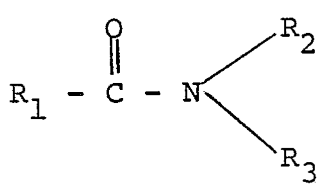

wherein $R_1$ is selected from the group consisting of non-linear $C_{10}$-$C_{22}$ fatty alkyls, $R_2$ is H or a $C_1$-$C_4$ hydroxyalkyl and $R_3$ is a $C_1$-$C_4$ hydroxyalkyl provided that if $R_1$ is a linear $C_{10}$-$C_{22}$ fatty alkyl, then $R_2$ is H to enhance clarity and gel strength.

2. The composition of claim 1, characterized in that water is present in amounts from about 60 to 90% by weight of said composition.

3. The composition of claim 1 or 2, characterized in that said water-soluble soap is present in amounts of from 5 to 20% by weight of said composition.

4. The composition of claim 1, 2 or 3, characterized in that said post-foaming agent is present in amounts from about 0.5 - 10% by weight of said composition.

5. The composition of any of claims 1 to 4, characterized in that said polymer gelling agent is present in amounts from about 0.1 to 5% by weight of said composition.

6. The composition of any of claims 1 to 5, characterized in that said alkanolamide is present in amounts from about 1 to 6% by weight of said composition.

7. The composition of claim 3, characterized in that said soap is a stearate or palmitate soap or an amine salt.

8. The composition of claim 7, characterized in that said amine salt is a triethanolamine salt.

9. The composition of claim 4, characterized in that said post-foaming agent is selected from the group consisting of butane, pentane, isobutane, isopentane, hexane, isomerized hexane, and mixtures thereof.

10. The composition of claim 5, characterized in that said gelling aid is hydroxyethyl cellulose or is selected from the group consisting of polyvinyl pyrrolidone, polyvinyl pyrrolidone related polymers and mixtures thereof or is a polysaccharide polymer.

11. The composition of claim 10, characterized in that said polysaccharide polymer is xanthan gum.

12. The composition of any of claims 1 to 11, characterized in that $R_1$ is a $C_{10}$-$C_{22}$ alkenyl.

13. The composition of any of claims 1 to 11, characterized in that $R_1$ is a non-linear $C_{10}$-$C_{22}$ alkyl.